# EUROPEAN PATENT APPLICATION

(11) **EP 1 298 223 A2**
(43) Date of publication of application: **02.04.2003**
(21) Application number: 02256379.5
(22) Date of filing: 16.09.2002
(51) Int. Cl.: C12Q 1/68, A61K 31/00, A61K 38/28

(54) **Methods related to the A-C repeat Z-sequence upstream from the aldose reductase gene**

(30) Priority: 28.09.2001 US 325927 P
(71) Applicant: Pfizer Products Inc., Groton, Connecticut 06340 (US)
(72) Inventor: Fryburg, David Albert, Pfizer Global, Groton, Connecticut 06340 (US); Klioze, Solomon Samuel, Pfizer Global, Groton, Connecticut 06340 (US); Milos, Patrice Marie, Pfizer Global, Groton, Connecticut 06340 (US); Oates, Peter Joseph, Pfizer Global, Groton, Connecticut 06340 (US)
(74) Representative: Hayles, James Richard

(57) **Abstract**

This invention relates to methods of characterizing subjects and methods of treatment or prevention of diseases and pathological conditions relating to the polymorphic A-C repeat sequence located approximately 2.1 kb upstream from the aldose reductase gene.

## Description

### FIELD OF THE INVENTION

This invention relates to methods of characterizing subjects and methods of treatment or prevention of diseases and pathological conditions relating to the polymorphic A-C repeat sequence located approximately 2.1 kb upstream from the aldose reductase gene.

### BACKGROUND OF THE INVENTION

The enzyme aldose reductase is involved in catalyzing the reduction of hexoses, such as glucose and galactose, to their corresponding polyols, such as sorbitol and galactitol. Certain diabetic complications, such as diabetic neuropathy, diabetic nephropathy and diabetic retinopathy, are believed to be associated with the accumulation of such polyols in the cells of affected tissues or with metabolic flux through the aldose reductase enzyme.

Shah, et al. J. Clin. Endocr. Metab., 82: 2294-2298, 1997, reported that aldose reductase gene expression, as measured by levels of mRNA, is higher in peripheral blood mononuclear cells of patients with insulin-dependent diabetes mellitus (IDDM) with diabetic nephropathy than in IDDM patients without diabetic nephropathy.

Ko, et al., Diabetes, 44(7): 727-732, 1995, identified a polymorphic A-C repeat sequence located 2.1 kb upstream from the transcription start site of the aldose reductase gene. Ko, et al. further identified seven alleles of the repeat sequence, having 21, 22, 23, 24, 25, 26 or 27 A-C repeats. The allele having 24 dinucleotide repeats is designated the "Z allele". Ko, et al. found that the Z-2 allele (i.e., having 23 A-C repeats) is associated with early onset of retinopathy in Chinese patients with non-insulin-dependent diabetes mellitus (NIDDM).

Heesom, et al., Diabetes, 46(2): 287-291, 1997, reports that in British Caucasian patients with IDDM, there was an increase in the frequency of the Z-2 allele in patients with nephropathy and an increase in the frequency of Z/Z-2 genotype.

Heesom, et al., J. Neurol Neurosurgry. & Psych, 64(2): 213-216, (1998), reports a decrease in the frequency of the Z+2 allele and a decrease in the Z/Z+2 genotype in patients with neuropathy as compared with patients without neuropathy.

Shah, et al., J. Clin. Endocr. Metab., 83: 2886-2891, 1998, identified 11 alleles of the A-C repeat sequence ranging from Z-12 to Z+8. The 1998 article found that among diabetic patients, aldose reductase messenger RNA levels are higher in those who are heterozygous or homozygous for the Z-2 allele compared with those lacking the Z-2 allele.

U.S. Patent 6,074,822 discloses methods for testing cells to determine whether a human diabetic patient has an abnormal aldose reductase RNA expression phenotype by isolating cells from the patient, exposing the cells to glucose and determining the level of aldose reductase RNA. The patent also discloses methods of treatment of a diabetic patient having an abnormal aldose reductase RNA expression phenotype by testing the cells of the patient according to the disclosed method and treating the patient with an inhibitor of aldose reductase.

Commonly assigned U.S. Patent 4,939,140 discloses the aldose reductase inhibitor, zopolrestat, and its use in the treatment of complications arising from diabetes mellitus.

### SUMMARY OF THE INVENTION

One aspect of this invention is characterization methods comprising:
determining the value of n of the (A-C)ₙ repeat Z sequence associated with each allele of the aldose reductase gene of a subject; and
characterizing said subject as having the attribute of preferentially benefiting from the administration of an agent for treatment or prevention of a disease or pathological condition that is mediated by having at least one allele of said Z sequence wherein the value of n is less than 24, with the proviso that the value of n of at least one allele of said subject is determined to be less than 24.

Another aspect of this invention is characterization methods comprising:
determining the value of n of the (A-C)ₙ repeat Z sequence associated with each allele of the aldose reductase gene of a subject; and
characterizing said subject as having the attribute of being likely to develop a disease or pathological condition that is mediated by having at least one allele of said Z sequence wherein the value of n is less than 24, with the proviso that the value of n of at least one allele of said subject is determined to be less than 24.

A further aspect of this invention is methods of treatment or prevention of complications associated with diabetes mellitus comprising administering to a subject an agent for treatment or prevention of a disease or pathological condition that is mediated by having at least one allele of the (A-C)ₙ repeat Z sequence associated with the aldose reductase gene wherein the value of n is less than 24, wherein said subject has been characterized as having at least one allele of said Z sequence wherein n is less than 24.

In a preferred embodiment of this invention, said subject has a disease or pathological condition that is mediated by having at least one allele of said Z sequence wherein n is less than 24.

In another preferred embodiment of this invention, said disease or pathological condition is a complication related to diabetes mellitus. In a more preferred embodiment, said disease or pathological condition is selected from arteriosclerosis, diabetic cardiomyopathy, cataracts, foot ulcers, diabetic macroangiopathy, diabetic microangiopathy, diabetic nephropathy, diabetic retinopathy and diabetic neuropathy. In an even more preferred embodiment, said disease or pathological condition is selected from diabetic nephropathy, diabetic retinopathy and diabetic neuropathy, preferably diabetic neuropathy.

In a further preferred embodiment of this invention the values of n of both alleles of said subject are determined to be less than 24.

In an additional preferred embodiment of the invention, said agent is selected from insulin, an insulin secretion stimulating sulfonylurea compound, a glycogen phosphorylase inhibitor (GPI), a biguanide hepatic glucose output inhibitor, a thiazolidinedione antidiabetic agent, an alpha-glucosidase inhibitor, a protein tyrosine phosphatase-1B (PTP-1B) inhibitor, a dipeptidyl peptidase IV (DPPIV) inhibitor, a glycogen synthase kinase-3 beta (GSK-3β) inhibitor, a peroxisome proliferator-activated receptor gamma (PPARγ) agonist and a glucagon receptor antagonist.
In another preferred embodiment of the invention, said agent is selected from a selective serotonin reuptake inhibitor (SSRI), a 3-hydroxy-3-methylglutaryl coenzyme A reductase inhibitor (a statin), a γ-aminobutyric acid (GABA) agonist, an angiotensin converting enzyme (ACE) inhibitor, an angiotensin-II (A-II) receptor antagonist, a phosphodiesterase type 5 (PDE-5) inhibitor, a polyol pathway inhibitor, a sorbitol dehydrogenase inhibitor (SDI) and an aldose reductase inhibitor (ARI). In a more preferred embodiment, said agent is a polyol pathway inhibitor. In an even more preferred embodiment, said polyol pathway inhibitor is selected from an aldose reductase inhibitor and a sorbitol dehydrogenase inhibitor.

For the embodiments of the invention wherein said polyol pathway inhibitor is a sorbitol dehydrogenase inhibitor, said sorbitol dehydrogenase inhibitor is preferably a compound of Formula A 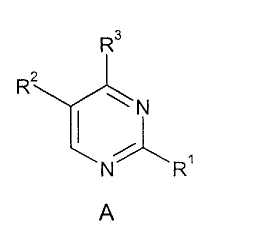 prodrugs thereof, or pharmaceutically acceptable salts thereof,
wherein R¹, R², and R³ are as described in International Patent Application publication number WO 00/59510.

For the embodiments of the invention wherein said polyol pathway inhibitor is an aldose reductase inhibitor, said aldose reductase inhibitor is selected from lindolrestat, fidarestat, epalrestat, zenarestat, ponalrestat, tolrestat, zopolrestat and a compound of Formula I 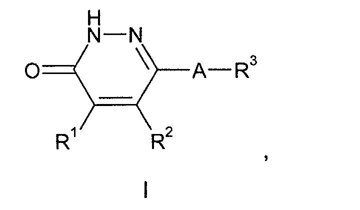 or a prodrug of said aldose reductase inhibitor or a pharmaceutcally acceptable salt of said aldose reductase inhibitor or said prodrug,
wherein:
A is S, SO or SO₂;
R¹ and R² are each independently hydrogen or methyl;
R³ is Het¹, -CHR⁴Het¹ or NR⁶R⁷;
R⁴ is hydrogen or (C₁-C₃)alkyl;
R⁶ is (C₁-C₆)alkyl, aryl or Het²;
R⁷ is Het³;
Het¹ is pyridyl, pyrimidyl, pyrazinyl, pyridazinyl, quinolyl, isoquinolyl, quinazolyl, quinoxalyl, phthalazinyl, cinnolinyl, naphthyridinyl, pteridinyl, pyrazinopyrazinyl, pyrazinopyridazinyl, pyrimidopyridazinyl, pyrimidopyrimidyl, pyridopyrimidyl, pyridopyrazinyl, pyridopyridazinyl, pyrrolyl, furanyl, thienyl, imidazolyl, oxazolyl, thiazolyl, pyrazolyl, isoxazolyl, isothiazolyl, triazolyl, oxadiazolyl, thiadiazolyl, tetrazolyl, indolyl, benzofuranyl, benzothienyl, benzimidazolyl, benzoxazolyl, benzothiazolyl, indazolyl, benzisoxazolyl, benzisothiazolyl, pyrrolopyridyl, furopyridyl, thienopyridyl, imidazolopyridyl, oxazolopyridyl, thiazolopyridyl, pyrazolopyridyl, isoxazolopyridyl, isothiazolopyridyl, pyrrolopyrimidyl, furopyrimidyl, thienopyrimidyl, imidazolopyrimidyl, oxazolopyrimidyl, thiazolopyrimidyl, pyrazolopyrimidyl, isoxazolopyrimidyl, isothiazolopyrimidyl, pyrrolopyrazinyl, furopyrazinyl, thienopyrazinyl, imidazolopyrazinyl, oxazolopyrazinyl, thiazolopyrazinyl, pyrazolopyrazinyl, isoxazolopyrazinyl, isothiazolopyrazinyl, pyrrolopyridazinyl, furopyridazinyl, thienopyridazinyl, imidazolopyridazinyl, oxazolopyridazinyl, thiazolopyridazinyl, pyrazolopyridazinyl, isoxazolopyridazinyl or isothiazolopyridazinyl; Het¹ is optionally substituted with up to a total of four substituents each independently selected from halo, formyl, (C₁-C₆)alkoxycarbonyl, (C₁-C₆)alkylenyloxycarbonyl, (C₁-C₄)alkoxy-(C₁-C₄)alkyl, C(OH)R¹²R¹³, (C₁-C₄)alkylcarbonylamido, (C₃-C₇)cycloalkylcarbonylamido, phenylcarbonylamido, benzyl, phenyl, naphthyl, imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy, (C₁-C₄)alkylsulfenyl, (C₁-C₄)alkylsulfonyl, (C₃-C₇)cycloalkyl, (C₁-C₆)alkyl optionally substituted with up to three fluoro, or (C₁-C₄)alkoxy optionally substituted with up to five fluoro; said benzyl, phenyl, naphthyl, imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy, in the definition of substituents for Het¹ are optionally substituted with up to three substituents independently selected from hydroxy, halo, hydroxy-(C₁-C₄)alkyl, (C₁-C₄)alkoxy-(C₁-C₄)alkyl, (C₁-C₆)alkylsulfenyl, (C₁-C₆)alkylsulfinyl, (C₁-C₆)alkylsulfonyl, (C₁-C₆)alkyl optionally substituted with up to five fluoro, and (C₁-C₄)alkoxy optionally substituted with up to five fluoro; said imidazolyl, oxazolyl, isoxazolyl, thiazolyl and pyrazolyl in the definition of substituents for Het¹ are optionally substituted with up to two substituents independently selected from hydroxy, halo, C₁-C₄)alkyl, hydroxy-(C₁-C₄)alkyl, (C₁-C₄)alkoxy-(C₁-C₄)alkyl, C₁-C₄)alkyl-phenyl optionally substituted in the phenyl portion with one Cl, Br, OMe, Me or SO₂-phenyl wherein said SO₂-phenyl is optionally substituted in the phenyl portion with one Cl, Br, OMe, Me, (C₁-C₄)alkyl optionally substituted with up to five fluoro, or (C₁-C₄)alkoxy optionally substituted with up to three fluoro;
   R¹² and R¹³ are each independently hydrogen or (C₁-C₄)alkyl;
Het² and Het³ are each independently imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy; Het² and Het³ are each independently optionally substituted with up to a total of four substituents each independently selected from halo, formyl, (C₁-C₆)alkoxycarbonyl, (C₁-C₆)alkylenyloxycarbonyl, (C₁-C₄)alkoxy-(C₁-C₄)alkyl, C(OH)R¹⁸R¹⁹, (C₁-C₄)alkylcarbonylamido, (C₃-C₇)cycloalkylcarbonylamido, phenylcarbonylamido, phenyl, naphthyl, imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy, (C₁-C₄)alkylsulfenyl, (C₁-C₄)alkylsulfonyl, (C₃-C₇)cycloalkyl, (C₁-C₄)alkyl optionally substituted with up to three fluoro or (C₁-C₄)alkoxy optionally substituted with up to five fluoro; said phenyl, naphthyl, imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy, in the definition of substituents for Het² and Het³ are optionally substituted with up to three substituents independently selected from hydroxy, halo, hydroxy-(C₁-C₄)alkyl, (C₁-C₄)alkoxy-(C₁-C₄)alkyl, (C₁-C₄)alkyl optionally substituted with up to five fluoro and (C₁-C₄)alkoxy optionally substituted with up to five fluoro; said imidazolyl, oxazolyl, isoxazolyl, thiazolyl and pyrazolyl in the definition of substituents for Het² and Het³ are optionally substituted with up to two substituents independently selected from hydroxy, halo, hydroxy-(C₁-C₄)alkyl, (C₁-C₄)alkoxy-(C₁-C₄)alkyl, (C₁-C₄)alkyl optionally substituted with up to five fluoro and (C₁-C₄)alkoxy optionally substituted with up to three fluoro; and
   R¹⁸ and R¹⁹ are each independently hydrogen or (C₁-C₄)alkyl;
   provided that when R³ is NR⁶R⁷, then A is SO₂. In a more preferabed embodiment, said aldose reductase inhibitor is zopolrestat, a compound of Formula I, a prodrug thereof or a pharmaceutcally acceptable salt thereof. In an even more preferred embodiment said aldose reductase inhibitor is zopolrestat, a prodrug thereof or a pharmaceutcally acceptable salt thereof. In another even more preferred embodiment said aldose reductase inhibitor is selected from: 6-(benzofuran-2-sulfonyl)-2H-pyridazin-3-one; 6-(5,7-dichloro-benzofuran-2-sulfonyl)-2H-pyridazin-3-one; 6-(5-chloro-benzofuran-2-sulfonyl)-2H-pyridazin-3-one; 6-(3-methyl-benzofuran-2-sulfonyl)-2H-pyridazin-3-one; 6-(5-trifluoromethyl-3-methyl-benzofuran-2-sulfonyl)-2H-pyridazin-3-one; 6-(5-fluoro-3-methyl-benzofuran-2-sulfonyl)-2H-pyridazin-3-one; 6-(5-chloro-3-methyl-benzothiophene-2-sulfonyl)-2H-pyridazin-3-one; 6-(5-chloro-3-methyl-benzofuran-2-sulfonyl)-2H-pyridazin-3-one; 6-benzothiophene-2-sulfonyl)-2H-pyridazin-3-one; 6-(3-[4-fluorophenyl]-benzofuran-2-sulfonyl)-2H-pyridazin-3-one; 6-(5-chloro-3-ethyl-benzofuran-2-sulfonyl)-2H-pyridazin-3-one; 6-(5-chloro-3-phenyl-2-sulfonyl)-2H-pyridazin-3-one; and 6-(5-methyl-benzofuran-2-sulfonyl)-2H-pyridazin-3-one, a prodrug thereof, or a pharmaceutically acceptable salt thereof.

In a further preferred embodiment of the invention, said subject is a mammal, preferably a human.

In one preferred embodiment of the characterization aspects of this invention, the characterizing of said subject comprises recording the identity and said attribute of said subject in an information recording media, preferably a recording media is selected from magnetic media, optical media and paper media.

The term "Z sequence" refers to the polymorphic A-C repeat sequence located approximately 2.1 kb upstream from the transcription start site of the aldose reductase gene.

The term "diabetes", as used herein, refers to diabetes mellitus (i.e., does not include diabetes insipidus). The term is meant to include Type I diabetes mellitus, also known as insulin dependent diabetes mellitus (IDDM), and Type II diabetes mellitus, also known as non-insulin dependent diabetes mellitus (NIDDM).

Diseases or pathological conditions mediated by having a Z sequence wherein the number of A-C repeats is less 24 (i.e., a "short Z sequence") is defined for the purposes herein to include: (a) diseases or conditions having a direct causal relationship to the short Z sequence; (b) diseases or conditions having an indirect causal relationship to the short Z sequence; and (c) diseases or conditions having no direct or indirect causal connection between the disease or pathological condition and the short Z sequence, but wherein the existence of the short Z sequence is indicative of such diseases or conditions. A direct causal connection includes conditions or diseases directly caused by the short sequence by itself or in combination with one or more other factors. An indirect causal connection includes one or more effects caused by the short sequence by itself or in combination with one or more other factors which do not directly cause the condition(s) or disease(s), but that cause one or more conditions, events or occurrences which ultimately result in the condition(s) or disease(s).

The expression "pharmaceutically acceptable salts" includes both pharmaceutically acceptable acid addition salts and pharmaceutically acceptable cationic salts, where appropriate. The expression "pharmaceutically-acceptable cationic salts" is intended to include, but is not limited to, such salts as the alkali metal salts, (e.g., sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to include, but is not limited to, such salts as the hydrochloride, hydrobromide, sulfate, hydrogen sulfate, phosphate, hydrogen phosphate, dihydrogenphosphate, acetate, succinate, citrate, methanesulfonate (mesylate) and p-toluenesulfonate (tosylate) salts. A particularly preferred salt is the sodium salt. Descriptions of compounds appearing herein which include the phrase "prodrugs thereof or pharmaceutically acceptable salts thereof" or a substantially similar phrase are meant to include both pharmaceutically acceptable salts of the applicable compounds as well as pharmaceutically acceptable salts of such prodrugs.

The expression "prodrug" refers to a compound that is a drug precursor which, following administration, releases the drug in vivo via some chemical or physiological process (e.g., a prodrug on being brought to the physiological pH or through enzyme action is converted to the desired drug form).

### DETAILED DESCRIPTION OF THE INVENTION

As reported by Graham, et al., J. Biol. Chem., 266:6872-6877, 1991, the aldose reductase gene extends over approximately 18 kb of chromosome 7 at region 7q35 and is transcribed into a 1,384 nucleotide mRNA, excluding the poly(A) tail. The polymorphic A-C repeat region used to characterize the genotype of subjects for the methods of this invention is located 2.1 kb upstream from the transcription start site of the aldose reductase gene.

Eleven alleles of the A-C repeat region have been identified *(see* Shah, et al., J. Clin. Endocr. Metab., 83: 2886-2891, 1998) ranging in number of A-C repeats from between 18 to 28 dinucleotides. Genotyping of subjects for the methods of the invention may be performed using genomic DNA isolated from whole blood. The repeat sequence region is amplified by polymerase chain reaction (PCR) techniques using a sense and an anti-sense primer described in Shah, et al., J. Clin. Endocr. Metab., 83: 2886-2891, 1998. The amplified region is 138 bp long for the allele having 24 dinucleotide A-C repeats. A sequence of the 138 bp region is found in Figure 2 of Ko, et al., Diabetes, 44(7): 727-732, 1995. The PCR product may be resolved by electrophoresis on a polyacrylamide gel.

The present invention includes characterization methods and methods of treatment or prevention related to diseases or pathological conditions that are mediated by having at least one allele of the (A-C)ₙ repeat Z sequence associated with the aldose reductase gene wherein the value of n is less than 24. Methods of identifying such diseases or conditions are well known to those skilled in the art. For example, Ko, et al., Diabetes, 44(7): 727-732, 1995, describes a study comparing the genotype of a group of diabetic patients with retinopathy against the genotype of a group of diabetic patients without retinopathy thereby finding that diabetic retinopathy is mediated by having the Z sequence wherein n is less than 24. Likewise Heesom, et al., Diabetes, 46(2): 287-291, 1997, describes using similar methods to find that diabetic nephropathy is mediated by having the Z sequence wherein n is less than 24. It will be apparent to those skilled in the art that methods similar to those described in Ko and Heesom may be employed to identify other diseases or conditions that are mediated by having at least one allele of the (A-C)ₙ repeat Z sequence associated with the aldose reductase gene wherein the value of n is less than 24.

In one embodiment of the present invention, the diseases or conditions that are mediated by having at least one allele of the (A-C)ₙ repeat Z sequence associated with the aldose reductase gene wherein the value of n is less than 24 are complications associated with diabetes. Such complications include arteriosclerosis, diabetic cardiomyopathy, cataracts, foot ulcers, diabetic macroangiopathy, diabetic microangiopathy, diabetic nephropathy, diabetic retinopathy and diabetic neuropathy. This list is not intended to be exhaustive. Other complications associated with diabetes that are mediated by having at least one allele of the aldose reductase associated Z sequence wherein n is less than 24 will be apparent to those with skill in the art and are included within the scope of this invention.

In another embodiment of the present invention, the characterization methods and methods of treatment or prevention relate to the administration of an agent for prevention or treatment of a disease or pathological condition that is mediated by having at least one allele of said Z sequence wherein the value of n is less than 24 (hereafter may be referred to as the "Agent(s)"). Such Agents will be known to those skilled in the art in light of this disclosure or may be readily identified by methods known in the art. For example, agents that inhibit the polyol pathway have been found to prevent or suppress diabetic associated retinopathy (Robison, et al., Ophthalmol. Vis. Sci., 30:2285-2292, 1989), cataracts (Dvornik, et al., Science, 182:1146-1148, 1973) and neuropathy (Green, et al., Neurology, 53:580-591, 1999).

Other Agents include ones that are effective in lowering or regulating glucose levels. Studies have shown that controlling blood glucose levels may delay the onset and progression of certain conditions related to diabetes (N. Engl. J. Med., 329:977-986, 1993). While not wishing to be bound by any particular theory or mechanism, it has been proposed that under hyperglycemic conditions the polyol, sorbitol, may accumulate as a product of the polyol pathway. Associated with the accumulation of sorbitol is a decrease in NADPH, myoinositol, Na⁺ -K⁺ dependent ATPase and an increase in NADH/NAD⁺. The accumulation of sorbitol and depletion of NADPH, myoinositol and Na⁺ -K⁺ dependent ATPase leads to the conditions associated with diabetes. Winegrad, Diabetes, 36:396-406, 1987; Williamson, et al., Diabetes, 42:801-813, 1993.

The Agents may include insulin, insulin secretion stimulating sulfonylurea compounds, glycogen phosphorylase inhibitors (GPI), biguanide hepatic glucose output inhibitors, thiazolidinedione antidiabetic agents, alpha-glucosidase inhibitors, protein tyrosine phosphatase-1B (PTP-1B) inhibitors, dipeptidyl peptidase IV (DPPIV) inhibitors, glycogen synthase kinase-3 beta (GSK-3β) inhibitors, peroxisome proliferator-activated receptor gamma (PPARγ) agonists, glucagon receptor antagonists, selective serotonin reuptake inhibitors (SSRI's), 3-hydroxy-3-methylglutaryl coenzyme A reductase inhibitors (statins), γ-aminobutyric acid (GABA) agonists, angiotensin converting enzyme (ACE) inhibitors, angiotensin-II (A-II) receptor antagonists, phosphodiesterase type 5 (PDE-5) inhibitors, polyol pathway inhibitors, sorbitol dehydrogenase inhibitors (SDI) and aldose reductase inhibitors (ARI).

The Agents may include any protein tyrosine phosphatase-1B (PTP-1B) inhibitor. The term protein tyrosine phosphatase-1B inhibitor refers to any agent that inhibits the enzyme protein tyrosine phosphatase-1B. PTP-1B is believed to inhibit the ability of insulin to reduce blood sugar levels

Exemplary PTP-1B inhibitors, assays for identifying such inhibitors and preferred dosage and methods of administration are disclosed in the following U.S. patents, International Patent Application publications and other publications (all of which are incorporated herein by reference):US 6,251936, US 6,221,902, US 6,057,316, US 6,001,867, US 5,753,687, WO 01/46203, WO 01/46204, WO 01/46206, WO 01/17516, WO 00/53583, WO 99/58518, WO 99/61435, WO 99/58521, WO 99/58522, WO 99/58514, WO 99/58520, WO 99/58519, WO 99/58511, WO 99/61410, WO 99/15529, Malamas, MS, et al. *J. Med. Chem.,* 43: 995-1010, 2000, Bleasdale, JE, et al., *Biochemistry,* 40: 5642-5654, 2001, Wrobel, J., et al., *J. Med. Chem.,* 42: 3199-3202, 1999, Malamas, MS, et al., *J. Med. Chem.,* 43(7): 1293-1310, 2000, Wang, et al., Med. Chem. Lett., 8 No. 4, 345-350, 1998, Taylor, et al., Bioorg. Med. Chem., 6, No. 9, 1457-1468, 1998, Andersen, et al., J. Biol. Chem., 275, No 10, 7101-7108, 2000, Iversen, et al., J. Biol. Chem., 275, No. 14, 10300-10307, 2000, Wrobel, et al., Bioorg.& Med. Chem. Lett., 10, 1535-1538, 2000 and Kees, J. Med. Chem., 39, 3920-3928, 1996.

The Agents may include any glucagon receptor antagonist. The term glucagon receptor antagonists refers to any agent that antagonizes the glucagon receptor, thus inhibiting the release of glucose induced by glucagon binding to the glucagon receptor. Such antagonism is readily determined by those skilled in the art according to assays known to those skilled in the art.

Exemplary glucagon receptor antagonists, assays for identifying such antagonists and preferred dosage and methods of administration are disclosed in the following U.S. patents, International Patent Application publications and other publications (all of which are incorporated herein by reference): US 6,218,431, US 5,138,090, WO 98/04528, WO 99/01423, WO 00/39088, WO 00/69810, WO 98/21957, WO 98/22109, WO 98/22108, WO 97/16442, Livingston, et al., *Diabetes* **1999,** *48* Suppl.1, 0862, Madsen, et al., *Journal of Medicinal Chemistry* **1998,** *41,* 5150-5157, de Laszlo, S.E., et al. *Biorganic & Medicinal Chemistry Letters* **1999,** *9,* 641-646, Chang, L.L., et al. *Biorganic & Medicinal Chemistry Letters* **2001,** *11,* 2549-2553, Cascieri, M.A., et al., *Journal of Biological Chemistry* **1999,** *274,* 8694-8697, Ling, A., et al., *Journal of Medicinal Chemistry* **2001,** *44,* 3141-3149 and Guillon, J., *et al. European Journal of Medicinal Chemistry* **1998,** *33*, 293-308.

The agents may include any glycogen synthase kinase-3 beta (GSK-3 β) antagonist. Exemplary GSK-3 β antagonists, assays for identifying such antagonists and preferred dosage and methods of administration are disclosed in the following U.S. patents, International Patent Application publications and other publications (all of which are incorporated herein by reference): US 6,057,286, WO 01/56567, WO 01/09106, WO 01/49709, WO 01/44246, WO 01/44206, WO 01/42224, WO 00/21927, WO 00/38675, WO 99/65897, WO 98/16528, Coghlan, M.P. et al., *Chemistry and Biology* **2000,** *7,* 793-803, Smith, D.G., et al., *Bioorganic & Medicinal Chemistry Letters* **2001,** *11,* 635-639, Cross, D.A.E., et al., *Journal of Neurochemistry* **2001**, 77, 94-102, Lochhead, P.A., et al., *Diabetes* **2001,** *50*, 1-10

The Agents for the invention may include alpha-glucosidase inhibitors. Any alpha-glucosidase inhibitor may be used as an Agent in the invention. Exemplary alpha-glucosidase inhibitors include acarbose (also known as Precose®) and miglitol (also known as Glyset™); and analogs, derivatives, prodrugs and pharmaceutically acceptable salts of those alpha-glucosidase inhibitors.

The Agents may include insulin secretion stimulating sulfonylurea compounds. Any insulin secretion stimulating sulfonylurea compound may be used as an Agent for the invention. Exemplary insulin secretion stimulating sulfonylurea compounds include glipizide, also known as Glucotrol® and Glucotrol XL®, glimepiride (also known as Amaryl®), glyburide and chlorpropamide (also known as Diabinese®); and analogs, derivatives, prodrugs and pharmaceutically acceptable salts thereof. A preferred insulin secretion stimulating sulfonylurea compounds is glipizide.

The Agents may include biguanide hepatic glucose output inhibitors. Any biguanide hepatic glucose output inhibitor may be used as an Agent in the practice of the invention. An exemplary biguanide is metformin, also known as Glucophage®.

The Agents may include PPARγ agonists, including thiazolidinedione and non-thiazolidinedione agents. PPARγ agonists increase insulin sensitivity in tissues important for insulin action such as adipose tissue, skeletal muscle, and liver.

Any PPARγ agonists may be used as an Agent in the practice of the invention. Exemplary PPARγ agonists include those described in the following U.S. Patent: US 4,340,605; US 4,342,771; US 4,367,234; US 4,617,312; US 4,687,777 and US 4,703,052; and analogs, derivatives, prodrugs and pharmaceutically acceptable salts thereof. Preferred PPARγ agonists include darglitazone, ciglitazone, englitazone, pioglitazone, also known as Actos®, and rosiglitazone, also known as Avandia® and BRL-49653. PPARγ agonists are preferably administered in amounts ranging from about 0.1 mg/day to about 100 mg/day in single or divided doses, preferably about 0.1 mg/day to about 50 mg/day for an average subject, depending upon the thiazolidinedione antidiabetic agent and the route of administration. However, some variation in dosage will necessarily occur depending on the condition of the subject being treated. The individual responsible for dosing will, in any event, determine the appropriate dose for the individual subject.

The Agents may include any dipeptidyl peptidase IV (DPP IV) inhibitors. The term DPP IV inhibitor refers to any agent which inhibits the enzyme dipeptidyl peptidase. Such inhibition is readily determined by those skilled in the art according to assays such as those disclosed in International Patent Application publication number WO 98/19998.

Exemplary DPP IV inhibitors include those disclosed in U.S. Patent Numbers US 6,124,305, US 6,110,949 and US 6,124,305, in International Patent Application publication numbers WO 01/34594, WO 99/61431, WO 98/19998, WO 97/40832 and WO 95/15309 and in KJL Augustyns, et al., 32 Eur. J. Med. Chem. 301-309 (1997); and analogs, derivatives, prodrugs and pharmaceutically acceptable salts thereof.

Preferred dosage and methods of administration are according to those provided in WO 01/34594 and WO 98/19998. Some variation in dosage may necessarily occur depending on the condition of the subject being treated. The individual responsible for dosing will, in any event, determine the appropriate dose for the individual subject.

The Agents may also comprise any selective serotonin reuptake inhibitor (SSRI). The term selective serotonin reuptake inhibitor refers to an agent which inhibits the reuptake of serotonin by afferent neurons. Such inhibition is readily determined by those skilled in the art according to standard assays such as those disclosed in U.S. 4,536,518 and other U.S. patents recited in the next paragraph.

Preferred SSRIs which may be used in accordance with this invention include femoxetine, which may be prepared as described in United States Patent No. 3,912,743; fluoxetine, which may be prepared as described in United States Patent No. 4,314,081; fluvoxamine, which may be prepared as described in United States Patent No. 4,085,225; indalpine, which may be prepared as described in United States Patent No. 4,064,255; indeloxazine, which may be prepared as described in United States Patent No. 4,109,088; milnacipran, which may be prepared as described in United States Patent No. 4,478,836; paroxetine, which may be prepared as described in United States Patent No. 3,912,743 or United States Patent No. 4,007,196; sertraline, which may be prepared as described in United States Patent No. 4,536,518; sibutramine, which may be prepared as described in United States Patent No. 4,929,629; and zimeldine, which may be prepared as described in United States Patent No. 3,928,369. Fluoxetine is also known as Prozac®. Sertraline hydrochloride, also known as Zoloft®, may be prepared as set forth in U.S. 4,536,518. Sibutramine is also known as Meridia®. SSRIs that may be used as Agents include analogs, derivatives, prodrugs and pharmaceutically acceptable salts of the SSRIs described above.

SSRIs are preferably administered in amounts ranging from about 0.01 mg/kg/day to about 500 mg/kg/day in single or divided doses, preferably about 10 mg to about 300 mg per day for an average subject, depending upon the SSRI and the route of administration. However, some variation in dosage will necessarily occur depending on the condition of the subject being treated. The individual responsible for dosing will, in any event, determine the appropriate dose for the individual subject.

The Agents may further comprise any 3-hydroxy-3-methylglutaryl coenzyme A (HMG-CoA) reductase inhibitor (statin). The term 3-hydroxy-3-methylglutaryl coenzyme A (HMG-CoA) reductase inhibitor refers to a pharmaceutical agent which inhibits the enzyme 3-hydroxy-3-methylglutaryl-coenzyme A (HMG-CoA) reductase. This enzyme is involved in the conversion of HMG-CoA to mevalonate, which is one of the steps in cholesterol biosynthesis. Such inhibition is readily determined according to standard assays well known to those skilled in the art.

Preferred statins which may be used in accordance with this invention include atorvastatin, disclosed in U.S. Patent No. 4,681,893, atorvastatin calcium, disclosed in U.S. Patent No. 5,273,995, cerivastatin, disclosed in U.S. 5,502,199, dalvastatin, disclosed in European Patent Application Publication No. 738,510 A2, fluindostatin, disclosed in European Patent Application Publication No. 363,934 A1, fluvastatin, disclosed in U.S. 4,739,073, lovastatin, disclosed in U.S. 4,231,938, mevastatin, disclosed in U.S. 3,983,140, pravastatin, disclosed in U.S. 4,346,227, simvastatin, disclosed in U.S. 4,444,784 and velostatin, disclosed in U.S. 4,448,784 and U.S. 4,450,171. Especially preferred 3-hydroxy-3-methylglutaryl coenzyme A reductase inhibitors include atorvastatin, atorvastatin calcium, also known as Lipitor®, lovastatin, also known as Mevacor®, pravastatin, also known as Pravachol®, and simvastatin, also known as Zocor®; and analogs, derivatives, prodrugs and pharmaceutically acceptable salts thereof.

Statins are preferably administered in amounts ranging from about 0.1 mg/kg to about 1000 mg/kg/day in single or divided doses, preferably about 1 mg/kg/day to about 200 mg/kg/day for an average subject, depending upon the statin and the route of administration. However, some variation in dosage will necessarily occur depending on the condition of the subject being treated. The individual responsible for dosing will, in any event, determine the appropriate dose for the individual subject.

The Agents may include any angiotensin converting enzyme (ACE) inhibitor. The term angiotensin converting enzyme inhibitor refers to a pharmaceutical agent which inhibits angiotensin converting enzyme activity. ACE is involved in the conversion of angiotensin I to the vasoconstrictor, angiotensin II. The activity of ACE inhibitors may readily be determined by methods known to those skilled in the art, including any of the standard assays described in the patents listed below.

Preferred ACE inhibitors include: alacepril, disclosed in U.S. Patent No. 4,248,883; benazepril, disclosed in U.S. Patent No. 4,410,520; captopril, disclosed in U.S. Patent Nos. 4,046,889 and 4,105,776; ceronapril, disclosed in U.S. Patent No. 4,452,790; delapril, disclosed in U.S. Patent No. 4,385,051; enalapril, disclosed in U.S. Patent No. 4,374,829; fosinopril, disclosed in U.S. Patent No. 4,337,201; imadapril, disclosed in U.S. Patent No. 4,508,727; lisinopril, disclosed in U.S. Patent No. 4,555,502; moexipril, disclosed in U.S. Patent No. 4,344,949; moveltopril, disclosed in Belgian Patent No. 893,553; perindopril, disclosed in U.S. Patent No. 4,508,729; quinapril, disclosed in U.S. Patent No. 4,344,949; ramipril, disclosed in U.S. Patent No. 4,587,258; spirapril, disclosed in U.S. Patent No. 4,470,972; temocapril, disclosed in U.S. Patent No. 4,699,905; and trandolapril, disclosed in U.S. Patent No. 4,933,361; and analogs, derivatives, prodrugs and pharmaceutically acceptable salts thereof.

ACE inhibitors are preferably administered in amounts ranging from about 0.01 mg/kg/day to about 500 mg/kg/day in single or divided doses, preferably about 10 mg to about 300 mg per day for an average subject, depending upon the ACE inhibitor and the route of administration. However, some variation in dosage will necessarily occur depending on the condition of the subject being treated. The individual responsible for dosing will, in any event, determine the appropriate dose for the individual subject.

The Agents may include any angiotensin-II receptor (A-II) antagonist. The term angiotensin-II receptor antagonist refers to a pharmaceutical agent that blocks the vasoconstrictor effects of angiotensin II by blocking the binding of angiotensin II to the AT₁ receptor found in many tissues, (e.g., vascular smooth muscle, adrenal gland). The activity of an A-II antagonist may readily be determined by methods known to those skilled in the art, including any of the standard assays described in the patents listed below.

Preferred A-II antagonists include: candesartan, which may be prepared as disclosed in U.S. Patent No. 5,196,444; eprosartan, which may be prepared as disclosed in U.S. Patent No. 5,185,351; irbesartan, which may be prepared as disclosed in U.S. Patent No. 5,270,317; losartan, which may be prepared as disclosed in U.S. Patent No. 5,138,069; and valsartan, which may be prepared as disclosed in U.S. Patent No. 5,399,578; and analogs, derivatives, prodrugs and pharmaceutically acceptable salts thereof. More preferred angiotensin-II receptor antagonists are losartan, irbesartan and valsartan.

A-II antagonists are preferably administered in amounts ranging from about 0.01 mg/kg/day to about 500 mg/kg/day in single or divided doses, preferably about 10 mg to about 300 mg per day for an average subject, depending upon the A-II antagonist and the route of administration. However, some variation in dosage will necessarily occur depending on the condition of the subject being treated. The individual responsible for dosing will, in any event, determine the appropriate dose for the individual subject.

The Agents may include any γ-aminobutyric acid (GABA) agonist. The term γ-aminobutyric acid agonist refers to a pharmaceutical agent that binds to GABA receptors in the mammalian central nervous system. GABA is the major inhibitory neurotransmitter in the mammalian central nervous system. The activity of a GABA agonist may readily be determined by methods known to those skilled in the art, including the procedures disclosed in Janssens de Verebeke, P. et al., Biochem. Pharmacol., 31, 2257-2261 (1982), Loscher, W., Biochem. Pharmacol., 31, 837-842, (1982) and/or Phillips, N. et al., Biochem. Pharmacol., 31, 2257-2261.

Preferred GABA agonists include: muscimol, which may be prepared as disclosed in U.S. 3,242,190; progabide, which may be prepared as disclosed in U.S. 4,094,992; riluzole, which may be prepared as disclosed in U.S. 4,370,338; baclofen, which may be prepared as disclosed in U.S. 3,471,548; gabapentin (Neurontin®), which may be prepared as disclosed in U.S. 4,024,175; vigabatrin, which may be prepared as disclosed in U.S. 3,960,927; valproic acid, which may be prepared as disclosed in Carraz et al., Therapie, 1965, 20, 419; tiagabine (Gabitril®), which may be prepared as disclosed in U.S. 5,010,090; lamotrigine (Lamictal®), which may be prepared as disclosed in U.S. 4,602,017; pregabalin, which may be prepared as disclosed in U.S. 6,028,214; phenytoin (Dilantin®), which may be prepared as disclosed in U.S. 2,409,754; carbamazepine (Tegretol®), which may be prepared as disclosed in U.S. 2,948,718; and topiramate (Topamax®) which may be prepared as disclosed in U.S. 4,513,006; and analogs, derivatives, prodrugs and pharmaceutically acceptable salts of those GABA agonists.

In general, in accordance with this invention, the GABA agonist used as the Agents will be administered in a dosage of about 4 mg/kg body weight of the subject to be treated per day to about 60 mg/kg body weight of the subject to be treated per day, in single or divided doses. However, some variation in dosage will necessarily occur depending upon the condition of the subject being treated. The person responsible for administration will, in any event, determine the appropriate dose for the individual subject. In particular, when used as the GABA agonist in this invention, pregabalin will be dosed at about 300 mg to about 1200 mg per day; gabapentin will be dosed at about 600 mg to about 3600 mg per day.

The Agents may include any glycogen phosphorylase inhibitor (GPI). The term glycogen phosphorylase inhibitor refers to any substance or agent or any combination of substances and/or agents which reduces, retards, or eliminates the enzymatic action of glycogen phosphorylase. Such actions are readily determined by those skilled in the art according to standard assays as described in U.S. Patent 5,988,463. U.S. Patent 5,988,463, PCT application publication WO 96/39384 and PCT application publication WO96/39385 exemplify GPI's that may be Agents.

GPIs are preferably administered in amounts ranging from about 0.005 mg/kg/day to about 50 mg/kg/day in single or divided doses, preferably about 0.1 mg/kg to about 15 mg/kg per day for an average subject, depending upon the GPI and the route of administration. However, some variation in dosage will necessarily occur depending on the condition of the subject being treated. The individual responsible for dosing will, in any event, determine the appropriate dose for the individual subject.

The Agents may include any sorbitol dehydrogenase inhibitor (SDI). The term sorbitol dehydrogenase inhibitor refers to any substance or agent or any combination of substances and/or agents which reduces, retards, or eliminates the enzymatic action of sorbitol dehydrogenase. Sorbitol dehydrogenase catalyzes the oxidation of sorbitol to fructose.

Exemplary SDIs include those disclosed in commonly assigned U.S. Patent No. 5,728,704, U.S. Patent No. 5,866,578 and PCT application publication WO 00/59510; and analogs, derivatives, prodrugs and pharmaceutically acceptable salts thereof. Preferred SDIs include compounds of Formula A 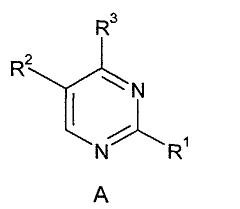 wherein R¹, R², and R³ are as described in WO 00/59510.

The activity of SDIs may be evaluated using the assays and methods disclosed in commonly assigned PCT application publication WO 00/59510 and other assays and methods known by those skilled in the art.

SDIs are preferably administered in amounts ranging from about 0.001 mg/kg/day to about 100 mg/kg/day in single or divided doses, preferably about 0.01 mg/kg to about 10 mg/kg per day for an average subject, depending upon the SDI and the route of administration. However, some variation in dosage will necessarily occur depending on the condition of the subject being treated. The person responsible for administration will, in any event, determine the appropriate dose for the individual subject.

The Agents may include any phosphodiesterase type 5 (PDE-5) inhibitor. The term phosphodiesterase type 5 inhibitor refers to any substance or agent or any combination of substances and/or agents which reduces, retards, or eliminates the enzymatic action of cyclic guanosine monophosphate (c-GMP)-specific PDE-5. Such actions are readily determined by those skilled in the art according to assays as described in PCT application publication WO 00/24745.

The following patent publications exemplify phosphodiesterase type 5 inhibitors which can be used as the Agents of this invention, and refer to methods of preparing those phosphodiesterase type 5 (PDE-5) inhibitors: PCT application publication WO 00/24745; PCT application publication WO 94/28902; European Patent application publication 0463756A1; European Patent application publication 0526004A1 and European Patent application publication 0201188A2. A preferred phosphodiesterase type 5 inhibitor is sildenafil (preferably sildenafil citrate, also known as Viagra®) which may be prepared as set forth in U.S. Patent No. 5,250,534 and 5,955,611. Exemplary PDE-5 inhibitors also include analogs, derivatives, prodrugs and pharmaceutically acceptable salts of the PDE-5 inhibitors listed above.

PDE-5 inhibitors are preferably administered in amounts ranging from about 5 mg/day to about 500 mg/day in single or divided doses, preferably about 10 mg/day to about 250 mg/day, for an average subject depending upon the PDE-5 inhibitor and the route of administration. However, some variation in dosage will necessarily occur depending on the condition of the subject being treated. The individual responsible for dosing will, in any event, determine the appropriate dose for the individual subject.

The Agents may include any aldose reductase inhibitor. The term aldose reductase inhibitor refers to compounds that inhibit the bioconversion of glucose to sorbitol catalyzed by the enzyme aldose reductase. Exemplary aldose reductase inhibitors include ponalrestat, disclosed in U.S. Patent 4,251,528, tolrestat, disclosed in U.S. Patent 4,600,724, epalrestat, disclosed in U.S. Patents 4,464,382, 4,791,126 and 4,831,045, zenarestat, disclosed in U.S. Patents 4,734,419, and 4,883,800 and zopolrestat disclosed in U.S. Patent 4,939,140. The foregoing U.S. patents are incorporated herein by reference. Exemplary aldose reductase inhibitors also include analogs, derivatives, prodrugs and pharmaceutically acceptable salts of the PDE-5 inhibitors listed above.

Other exemplary aldose reductase inhibitors include compounds of Formula I 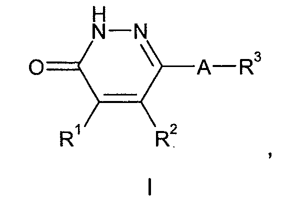 or a prodrug of said aldose reductase inhibitor or a pharmaceutcally acceptable salt of said aldose reductase inhibitor or said prodrug,
wherein:
A is S, SO or SO₂;
R¹ and R² are each independently hydrogen or methyl;
R³ is Het¹, -CHR⁴Het¹ or NR⁶R⁷;
R⁴ is hydrogen or (C₁-C₃)alkyl;
R⁶ is (C₁-C₆)alkyl, aryl or Het²;
R⁷ is Het³;
Het¹ is pyridyl, pyrimidyl, pyrazinyl, pyridazinyl, quinolyl, isoquinolyl, quinazolyl, quinoxalyl, phthalazinyl, cinnolinyl, naphthyridinyl, pteridinyl, pyrazinopyrazinyl, pyrazinopyridazinyl, pyrimidopyridazinyl, pyrimidopyrimidyl, pyridopyrimidyl, pyridopyrazinyl, pyridopyridazinyl, pyrrolyl, furanyl, thienyl, imidazolyl, oxazolyl, thiazolyl, pyrazolyl, isoxazolyl, isothiazolyl, triazolyl, oxadiazolyl, thiadiazolyl, tetrazolyl, indolyl, benzofuranyl, benzothienyl, benzimidazolyl, benzoxazolyl, benzothiazolyl, indazolyl, benzisoxazolyl, benzisothiazolyl, pyrrolopyridyl, furopyridyl, thienopyridyl, imidazolopyridyl, oxazolopyridyl, thiazolopyridyl, pyrazolopyridyl, isoxazolopyridyl, isothiazolopyridyl, pyrrolopyrimidyl, furopyrimidyl, thienopyrimidyl, imidazolopyrimidyl, oxazolopyrimidyl, thiazolopyrimidyl, pyrazolopyrimidyl, isoxazolopyrimidyl, isothiazolopyrimidyl, pyrrolopyrazinyl, furopyrazinyl, thienopyrazinyl, imidazolopyrazinyl, oxazolopyrazinyl, thiazolopyrazinyl, pyrazolopyrazinyl, isoxazolopyrazinyl, isothiazolopyrazinyl, pyrrolopyridazinyl, furopyridazinyl, thienopyridazinyl, imidazolopyridazinyl, oxazolopyridazinyl, thiazolopyridazinyl, pyrazolopyridazinyl, isoxazolopyridazinyl or isothiazolopyridazinyl; Het¹ is optionally substituted with up to a total of four substituents each independently selected from halo, formyl, (C₁-C₆)alkoxycarbonyl, (C₁-C₆)alkylenyloxycarbonyl, (C₁-C₄)alkoxy-(C₁-C₄)alkyl, C(OH)R¹²R¹³, (C₁-C₄)alkylcarbonylamido, (C₃-C₇)cycloalkylcarbonylamido, phenylcarbonylamido, benzyl, phenyl, naphthyl, imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy, (C₁-C₄)alkylsulfenyl, (C₁-C₄)alkylsulfonyl, (C₃-C₇)cycloalkyl, (C₁-C₆)alkyl optionally substituted with up to three fluoro, or (C₁-C₄)alkoxy optionally substituted with up to five fluoro; said benzyl, phenyl, naphthyl, imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy, in the definition of substituents for Het¹ are optionally substituted with up to three substituents independently selected from hydroxy, halo, hydroxy-(C₁-C₄)alkyl, (C₁-C₄)alkoxy-(C₁-C₄)alkyl, (C₁-C₆)alkylsulfenyl, (C₁-C₆)alkylsulfinyl, (C₁-C₆)alkylsulfonyl, (C₁-C₆)alkyl optionally substituted with up to five fluoro, and (C₁-C₄)alkoxy optionally substituted with up to five fluoro; said imidazolyl, oxazolyl, isoxazolyl, thiazolyl and pyrazolyl in the definition of substituents for Het¹ are optionally substituted with up to two substituents independently selected from hydroxy, halo, C₁-C₄)alkyl, hydroxy-(C₁-C₄)alkyl, (C₁-C₄)alkoxy-(C₁-C₄)alkyl, C₁-C₄)alkyl-phenyl optionally substituted in the phenyl portion with one Cl, Br, OMe, Me or SO₂-phenyl wherein said SO₂-phenyl is optionally substituted in the phenyl portion with one Cl, Br, OMe, Me, (C₁-C₄)alkyl optionally substituted with up to five fluoro, or (C₁-C₄)alkoxy optionally substituted with up to three fluoro;
   R¹² and R¹³ are each independently hydrogen or (C₁-C₄)alkyl;
Het² and Het³ are each independently imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy; Het² and Het³ are each independently optionally substituted with up to a total of four substituents each independently selected from halo, formyl, (C₁-C₆)alkoxycarbonyl, (C₁-C₆)alkylenyloxycarbonyl, (C₁-C₄)alkoxy-(C₁-C₄)alkyl, C(OH)R¹⁸R¹⁹, (C₁-C₄)alkylcarbonylamido, (C₃-C₇)cycloalkylcarbonylamido, phenylcarbonylamido, phenyl, naphthyl, imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy, (C₁-C₄)alkylsulfenyl, (C₁-C₄)alkylsulfonyl, (C₃-C₇)cycloalkyl, (C₁-C₄)alkyl optionally substituted with up to three fluoro or (C₁-C₄)alkoxy optionally substituted with up to five fluoro; said phenyl, naphthyl, imidazolyl, pyridyl, triazolyl, benzimidazolyl, oxazolyl, isoxazolyl, thiazolyl, oxadiazolyl, thiadiazolyl, tetrazolyl, thienyl, benzothiazolyl, pyrrolyl, pyrazolyl, quinolyl, isoquinolyl, benzoxazolyl, pyridazinyl, pyridyloxy, pyridylsulfonyl, furanyl, phenoxy, thiophenoxy, in the definition of substituents for Het² and Het³ are optionally substituted with up to three substituents independently selected from hydroxy, halo, hydroxy-(C₁-C₄)alkyl, (C₁-C₄)alkoxy-(C₁-C₄)alkyl, (C₁-C₄)alkyl optionally substituted with up to five fluoro and (C₁-C₄)alkoxy optionally substituted with up to five fluoro; said imidazolyl, oxazolyl, isoxazolyl, thiazolyl and pyrazolyl in the definition of substituents for Het² and Het³ are optionally substituted with up to two substituents independently selected from hydroxy, halo, hydroxy-(C₁-C₄)alkyl, (C₁-C₄)alkoxy-(C₁-C₄)alkyl, (C₁-C₄)alkyl optionally substituted with up to five fluoro and (C₁-C₄)alkoxy optionally substituted with up to three fluoro; and
R¹⁸ and R¹⁹ are each independently hydrogen or (C₁-C₄)alkyl; provided that when R³ is NR⁶R⁷, then A is SO₂.

Aldose reductase inhibitors of Formula I may be prepared according to **Scheme 1,**

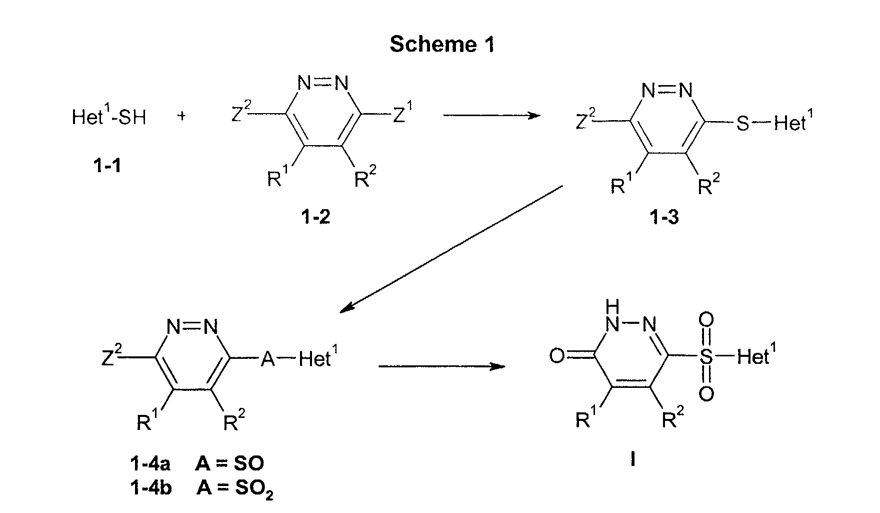

In **Scheme 1,** the aldose reductase inhibitors of Formula **I,** wherein R¹ and R² are as defined above and R³ is Het¹, can be prepared from the corresponding pyridazine of formula **1-2** and a heterocyclic thiol of formula **1-1.** A thiol **1-1,** in which R³ of the compounds of Formula I is Het¹, is reacted with a base such as an alkali metal (C₁-C₆)alkoxide in a (C₁-C₆) alkanol, to obtain the alkali metal salt of said thiol. Preferred alkali metal (C₁-C₆)alkoxides include, but are not limited to, sodium methoxide, sodium ethoxide and potassium t-butoxide. After evaporating the excess solvent, the resulting alkali metal salt of said thiol is refluxed with a compound of formula **1-2** wherein Z¹ and Z² are each independently selected from chloro, (C₁-C₆)alkoxy, phenyloxy or benzyloxy, said benzyloxy or phenyloxy being optionally substituted with one or two chloro or methyl groups in an aromatic hydrocarbon solvent or solvent system, for example, toluene, benzene or xylene. The reaction is allowed to stir overnight to obtain a compound of formula **1-3**. The reaction is usually conducted at ambient pressure and at the refluxing temperature of the solvent used. Compounds of formula **1-3** can also be prepared by reacting compounds **1-2,**
wherein R¹, R², Z¹ and Z² are as defined above with a compound of formula **1-1** in a reaction inert solvent such as a polar non-aqueous solvent containing an alkali or alkali earth metal hydride or an alkali or alkali earth (C₁-C₄)alkoxide. Preferred such solvents include, but are not limited to, acetonitrile and ether solvents such as diglyme, tetrahydrofuran (THF) and dimethylformamide (DMF). Preferred such alkali or alkali earth metal hydrides include, but are not limited to, sodium hydride. Preferred alkali or alkali earth metal (C₁-C₄)alkoxides include, but are not limited to, potassium t-butoxide. The preferred metal hydride is sodium hydride. A particularly preferred solvent is DMF. Compounds of formula **1-3** can also be prepared by reacting a compound of formula **1-1** with a compound of formula **1-2**, wherein the variables are as defined above, in a reaction inert solvent such as DMF, THF, diglyme or dioxane containing sodium carbonate, potassium carbonate, sodium bicarbonate or potassium bicarbonate. This reaction is usually conducted at ambient pressure and at temperatures between about 60°C and about 120°C. A compound of formula **1-3** can be oxidized to afford a sulfoxide or a sulfonyl compound of formula **1-4a** and/or **1-4b,** respectively. A preferred procedure is oxidation of a compound of formula **1-3** with 30% hydrogen peroxide in the presence or absence of an organic acid such as formic acid or acetic acid. Another preferred oxidation procedure involves the use of peracid in the corresponding organic acid as solvent. Yet another preferred procedure is oxidation of a compound of formula **1-3** with a peracid, for example meta-chloroperbenzoic acid (MCPBA), in a halocarbon solvent, for example, methylene chloride, chloroform or ethylene chloride. In any case, the reaction is conducted at ambient pressure and at temperatures between about 20°C and about-40°C with careful reaction monitoring to avoid formation of N-oxides by over-oxidation at the nitrogen atom. The oxidation reaction is usually complete within three to six hours and proceeds through sulfoxide **1-4a**, but occasionally may be complete prior to the passage of three hours, as determined by a person skilled in the art. If the reaction is conducted at between about 20°C and about 30°C, and is stopped at between one to three hours, sulfoxide **1-4a** can be isolated using separation procedures well known to a person skilled in the art. The resulting sulfone of formula **1-4b** can then be hydrolyzed with a mineral acid such as, but not limited to, concentrated hydrochloric acid with no solvent or in a reaction inert solvent such as an ether solvent, for example, dioxane, tetrahydrofuran or diethyl ether, to obtain a compound of Formula I. The hydrolysis reaction is generally conducted at ambient pressure and at the refluxing temperature of the solvent used.

The aldose reductase inhibitors of Formula I may also be prepared according to **Scheme 2,**

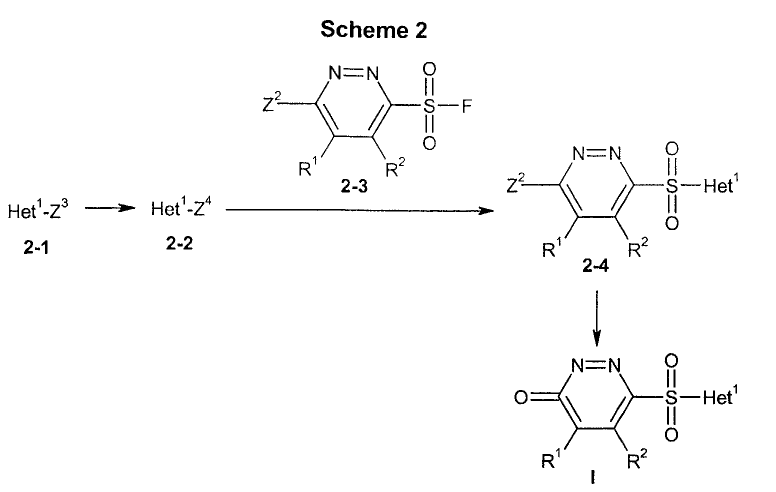

In **Scheme 2,** the aldose reductase inhibitors of Formula **I** may be prepared by reacting compounds of the formula Het¹-Z³ where Z³ is bromide, iodide or an acidic hydrogen with a suitable organometallic base to form compounds of the formula Het¹-Z⁴ wherein Z⁴ is the cation corresponding to the organometallic base. Het¹-Z⁴ may in turn may be reacted with a fluorosulfonyl pyridazine compound of the formula **2-3** to form a sulfonyl pyridazine of the formula **2-4** which may be hydrolyzed to form a compound of Formula I. In the case where Z³ is an acidic hydrogen, the hydrogen will be acidic enough such that said hydrogen is removable by reaction with a base such as, but not limited to, (C₁-C₆)alkyllithium, lithium diisopropylamide (LDA) or phenyl lithium. Thus, a compound of formula **2-1** in which Z³ is bromide, iodide or a hydrogen of sufficient acidity, is reacted with a base such as, but not limited to, (C₁-C₆)alkyllithium, lithium diisopropylamide (LDA) or phenyl lithium to prepare a compound of formula **2-2,** wherein Z⁴ is lithium. A hydrogen of sufficient acidity is a hydrogen that can be removed from Het¹-Z³ by the bases mentioned in the preceding sentence. The reaction is conducted in a reaction inert solvent such as an ether or a hydrocarbon solvent or a mixture of such solvents. Preferred solvents include, but are not limited to, diethyl ether, tetrahydrofuran, diglyme, benzene and toluene or mixtures thereof. The reaction is conducted at temperatures from about -78°C to about 0°C and at ambient pressure. A compound of formula **2-2** is reacted with a compound of formula **2-3** wherein Z² is chloro, (C₁-C₆)alkoxy, phenyloxy or benzyloxy, said phenyloxy or benzyloxy being optionally substituted with one or two chloro or methyl groups to form compounds of formula **2-4** wherein Z² is as defined above. The reaction is conducted in a reaction inert solvent such as an ether or a hydrocarbon solvent or a mixture of such solvents. Preferred solvents include, but are not limited to, diethyl ether, tetrahydrofuran, diglyme, benzene and toluene or mixtures thereof. The reaction is conducted at temperatures ranging from about-78°C to about 0°C and at ambient pressure. Compounds **2-4** are hydrolyzed to form compounds of Formula I as described above.

Also according to **Scheme 2,** compounds of formula **2-4** may be prepared by reacting a compound of formula **2-2** wherein Z⁴ is MgBr or Mgl using standard Grignard reaction conditions, e.g., by reacting a compound of formula **2-1** wherein Z³ is bromide or iodide with magnesium to form the compound of formula **2-2** which is reacted, preferably *in situ,* with a compound of formula **2-3** wherein Z² is as defined above. The reaction is generally conducted in a reaction inert solvent such as an ether or a hydrocarbon solvent or a mixture of such solvents. Preferred solvents include, but are not limited to, diethyl ether, tetrahydrofuran, diglyme, benzene and toluene or mixtures thereof. The reaction temperature ranges from about -10°C to about 40°C. Formation of the Grignard reagent of formula **2-2** may be readily accomplished according to methods well known to those skilled in the art.

The aldose reductase inhibitors of Formula I may also be prepared according to **Scheme 3**,

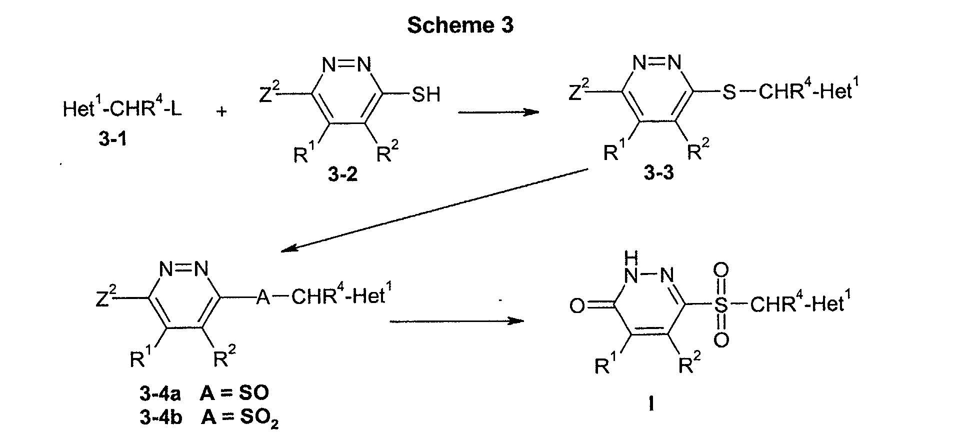

In **Scheme 3,** the aldose reductase inhibitors of Formula I wherein R¹, R², Z² and Het¹ are defined as described above and R³ is CHR⁴-Het¹ may be prepared by reacting a compound of the formula **3-1** with a compound of the formula **3-2** followed by further modification. Thus, a compound of the formula **3-1** wherein L is a leaving group such as chloro, bromo, iodo, methanesulfonyloxy, phenylsulfonyloxy wherein said phenyl of said phenylsulfonyloxy may be optionally substituted by one nitro, chloro, bromo or methyl is reacted with a compound of the formula **3-2,** wherein Z² is as described above, to form a compound of the formula **3-3.** The reaction is conducted in a reaction inert solvent such as methylene chloride, chloroform, diethyl ether, tetrahydrofuran, dioxane, acetonitrile or dimethylformamide at a temperature ranging from about room temperature to about 90°C. The reaction is conducted at ambient pressure. A compound of the formula **3-3** is then oxidized to form a sulfoxide or sulfonyl compound of the formula **3-4a** and/or **3-4b,** respectively, by reacting said compound of formula **3-3** with an oxidizing agent such as metachloroperbenzoic acid (MCPBA) in a reaction inert solvent or hydrogen peroxide in acetic acid. The sulfoxide of formula **3-4a** may be isolated by halting the oxidation reaction as described in **Scheme 1** above. When MCPBA is used, preferred reaction inert solvents include such solvents as methylene chloride and chloroform. The reaction is ordinarily performed at room temperature. When hydrogen peroxide is used as the oxidizing agent, the reaction is carried out as described above. Compounds of formula **3-4b** thus prepared may be hydrolyzed to form compounds of Formula I according to conditions described in Scheme 1 above.

The aldose reductase inhibitors of Formula I may also be prepared according to **Scheme 4**,

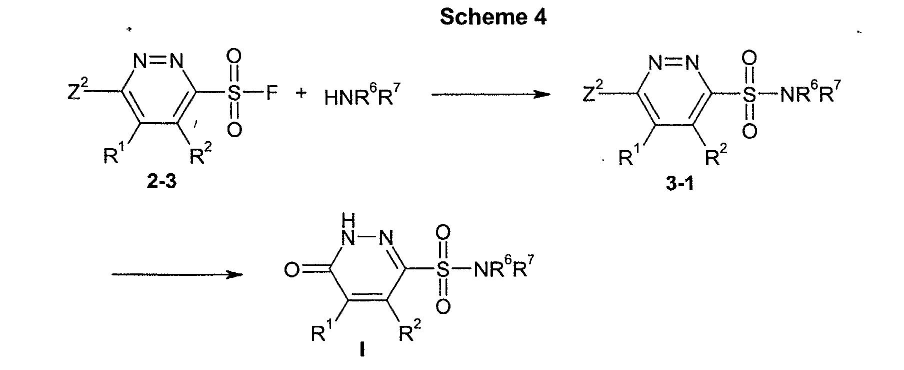

In **Scheme 4,** the aldose reductase inhibitors of Formula I wherein R¹, R² and Z are defined as set forth above and R³ is -NR⁶R⁷ may be prepared from compounds of formula **2-3.** Thus, a compound of formula **2-3** is reacted with an amine of the formula HNR⁶R⁷, wherein R⁶ and R⁷ are defined as set forth above, in the presence of excess HNR⁶R⁷ or a tertiary amine such as, but not limited to, triethyl amine or diisopropyl ethyl amine in a reaction inert solvent to form a compound of the formula **3-1**. Preferred reaction inert solvents for this reaction include, but are not limited to, methylene chloride, chloroform, diethyl ether, tetrahydrofuran and dioxane. The reaction is preferably conducted at a temperature ranging from about 0°C to about 100°C. Compounds of formula **3-1** thus prepared may be hydrolyzed to form compounds of Formula I as described above.

Pharmaceutically acceptable cationic salts of the aldose reductase inhibitors of Formula I may be readily prepared by reacting the free acid form of said compounds with an appropriate base, usually one equivalent, in a co-solvent. Typical bases are sodium hydroxide, sodium methoxide, sodium ethoxide, sodium hydride, potassium methoxide, potassium carbonate, sodium carbonate, magnesium hydroxide, calcium hydroxide, benzathine, choline, diethanolamine, piperazine and tromethamine. The salt is isolated by concentration to dryness or by addition of a non-solvent. In many cases, salts are preferably prepared by mixing a solution of the acid with a solution of a different salt of the cation (sodium or potassium ethylhexanoate, magnesium oleate), and employing a solvent (e.g., ethyl acetate) from which the desired cationic salt precipitates, or can be otherwise isolated by concentration and/or addition of a non-solvent. They can be further purified by crystallization from (C₁-C₆)alcoholic solvents such as methanol, ethanol or isopropanol or from ketonic solvents such as acetone, methyl ethyl ketone or methyl isobutyl ketone.

The acid addition salts of the aldose reductase inhibitors of Formula I may be readily prepared by reacting the free base form of said compounds with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent. They can be further purified by crystallization from (C₁-C₆)alcoholic solvents such as methanol, ethanol or isopropanol or from ketonic solvents such as acetone, methyl ethyl ketone or methyl isobutyl ketone.

Prodrugs of the aldose reductase inhibitors of Formula I may be formed by substituting a compound of Formula I at the 2-nitrogen position of the pyridazin-3-one ring as shown below: 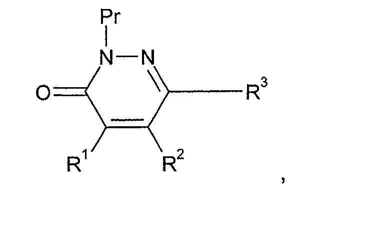 wherein Pr is (C₁-C₆)alkyl or benzyl. These prodrugs may be prepared by reacting a compound of Formula I with a compound of the formula Pr-X, wherein Pr is as defined above and X is bromo, chloro or iodo in the presence of a base such as, for example, sodium hydride or n-butyl lithium in a reaction inert solvent, such as, for example, dimethylformamide, tetrahydrofuran or ether. The reaction is generally carried out at temperatures ranging from about 0°C to about room temperature when using sodium hydride as the base. When using n-butyl lithium or a similar base, the reaction is generally carried out at temperatures ranging from about -60°C to about 0°C. Other processes for preparing such prodrugs will be readily apparent to the skilled person.

Inhibition of the polyol pathway by aldose reductase inhibitors is readily determined by those skilled in the art according to standard assays (J. Malone, Diabetes, 29:861-864, 1980. "Red Cell Sorbitol, an Indicator of Diabetic Control"). The amount of aldose reductase inhibitor that is administered per dose and the intervals between doses will depend upon the aldose reductase inhibitor being used, the type of pharmaceutical compositions being used, the characteristics of the subject being treated and the severity of the conditions, if any, being treated. Aldose reductase inhibitors are preferably administered in amounts ranging from about 0.001 mg/kg/day to about 1000 mg/kg/day in single or divided doses, preferably about 0.01 mg/kg to about 500 mg/kg per day for an average subject, depending upon the aldose reductase inhibitor and the route of administration. However, some variation in dosage will necessarily occur depending on the condition of the subject being treated. The person responsible for administration will, in any event, determine the appropriate dose for the individual subject. A preferred aldose reductase inhibitor is zopolrestat which is administered preferably at a dosage of between 250 mg and 500 mg per day.

The Agent is employed for the methods of this invention either alone or in combination with another Agent. The Agent(s) may be administered alone or with one or more pharmaceutically acceptable carriers, diluents or fillers. Pharmaceutical compositions containing the Agent(s) may be readily administered in a variety of dosage forms such as tablets, powders, lozenges, syrups, injectable solutions and so forth. These pharmaceutical compositions may, if so desired, contain additional ingredients such as flavorings, binders, excipients and the like. For the purposes of oral administration, tablets containing various excipients such as sodium citrate, calcium carbonate, and calcium diphosphate may be used along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate, and talc are often useful for tabletting purposes. Solid compositions of a similar type may also be used as fillers in soft and hard filled gelatin capsules. Preferred materials for this use include lactose or milk sugar and high molecular weight polyethylene glycols. When aqueous suspensions or elixirs are desired for oral administration, the Agent therein may be combined with various sweetening or flavoring agents, coloring matter or dyes and, if desired, emulsifying or suspending agents, together with diluents such as water, ethanol, propylene glycol, glycerin or various combinations thereof.

For parenteral administration, solutions of the Agent(s) useful in this invention, in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, the sterile aqueous media employed are all readily available by standard techniques known to those skilled in the art.

In one embodiment of the method of treatment or prevention aspects of this invention, subjects are further characterized as having diabetes mellitus. The World Health Organization (W.H.O.) has suggested criteria for diagnosing diabetes mellitus (W.H.O. 1980/85 Technical Report Series No. 646/727). Diabetes has also been characterized by the National Diabetes Data Group (see National Diabetes Data Group: Classification and diagnosis of diabetes mellitus and other categories of glucose intolerance, Diabetes, 28:1039-1044, 1979).

Diabetic neuropathy, a complication arising from diabetes mellitus, is an example of conditions that are mediated by having at least one allele of the (A-C)ₙ repeat Z sequence associated with the aldose reductase gene wherein the value of n is less than 24. Changes in composite neuroelectrophysiology (NE) parameter following administration of an Agent in subjects having at least one allele of the said Z sequence wherein n is less than 24 may be used to demonstrate the present invention. Such evaluation may be performed by measuring changes in nerve conduction velocity (NCV) in the following nerves: median motor, ulnar sensory, median sensory (both distal and proximal segments), peroneal motor and sural sensory. Testing is performed prior to the initiation of administration of the Agent and one or more times thereafter. An exemplary schedule for neuroelectrophysiology evaluation is prior to initiation of administration of the Agent and at weeks 12, 24 and 52 following initiation of administration.

Tables 1 and 2 illustrate the effect of administration of the aldose reductase inhibitor, zopolrestat, on NCV of subjects having one and two alleles of the Z sequence having less than 24 A-C repeats. The results are based upon a double-blind study in which subjects with painful, symmetrical polyneuropathy were treated with a placebo or zopolrestat (1000 mg/day) for up to 12 weeks. The NCV results from Tables 1 and 2 where obtained according to the protocol described in Example 2 of the General Experimental Procedures.

Composite NE parameter is calculated according to the technique described in O'Brien, P.C., Biometrics, 40, 1079-1087 (1984). For ease of interpretation, the standard composite NE parameter of O'Brien is slightly modified for Tables 1 and 2. The standard O'Brien method would give values of 0 to 1. For the modified O'Brien score, the standard O'Brien value is reduced by 0.5 to give a value in the range of - 0.5 to 0.5 with an expected value of 0. Values lower than 0 correspond to an overall composite NE decrease relative to the entire patient population, while values greater than 0 correspond to a relative increase.

The terms "1 short" and "2 short" as used in Tables 1 and 2 refers to the number of alleles found in each subject where n has a value of less than 24 for the (A-C)ₙ repeat Z sequence. "1 short" refers to subjects having only one allele wherein n is less than 24. "2 short" refers to subjects having two alleles wherein n is less than 24.

The comparison in Table 2 between the placebo group and the treatment group was performed using one-way Analysis of Variance (ANOVA).

**TABLE 1**

| **Placebo Group** | | | | | | | | | | | |
|---|---|---|---|---|---|---|---|---|---|---|---|
| Nerve | Group | | Baseline | | 12 Week Change | | | | | | |
| | | N | Mean | Median | Mean | Median | 95% CI | | SE | P-Value (2-sided) | |
| | | | | | | | | | | Within | between |
| | | | | | | | | | | (vs 0) | (1 vs 2) |
| | | | | | | | | | | | |
| **Peroneal Motor** | Combined | 28 | 40.61 | 39.47 | -0.94 | -0.60 | -2.38 | 0.51 | 0.70 | 0.19 | |
| | 1 Short | 9 | 39.24 | 38.81 | 1.00 | 0.10 | -2.02 | 4.03 | 1.31 | 0.47 | |
| | 2 Short | 19 | 41.18 | 39.47 | -1.86 | -0.72 | -3.47 | -0.24 | 0.77 | **0.03** | **0.06** |
| | | | | | | | | | | | |
| **Composite NE Parameter** (modified) | Combined | 30 | | | -0.05 | -0.05 | -0.11 | 0.00 | 0.03 | **0.04** | |
| | 1 Short | 10 | | | -0.02 | -0.03 | -0.15 | 0.10 | 0.05 | 0.66 | |
| | 2 Short | 20 | | | -0.07 | -0.05 | -0.13 | -0.01 | 0.03 | **0.02** | 0.42 |
| | | | | | | | | | | | |

**TABLE 2.**

| **Placebo vs Combined Treatment Groups** **(Includes comparison to the 250 and 500 mg zopolrestat dose)** | | | | | | | | |
|---|---|---|---|---|---|---|---|---|
| | | | 12 Week Change | | | | | |
| | | | | | | | P-Value (2-sided) | |
| | | | | | | | Placebo vs. Treatment | 1 short vs. 2 short |
| Nerve | Group | N | Mean Treatment Effect | 95% CI | | SE | | |
| | | | | | | | | |
| **Peroneal** **Motor** | Combined | 88 | 1.63 | 0.04 | 2.87 | 0.62 | **0.01** | **0.02** |
| | 1 Short | 26 | 0.22 | -2.26 | 2.70 | 1.20 | 0.86 | |
| | 2 Short | 62 | 2.35 | 0.97 | 3.72 | 0.69 | **<0.01** | |
| | | | | | | | | |
| **Composite** **NE Parameter** (modified) | Combined | 95 | 0.07 | 0.01 | 0.13 | 0.03 | **0.02** | 0.72 |
| | 1 Short | 28 | 0.04 | -0.10 | 0.17 | 0.06 | 0.58 | |
| | 2 Short | 67 | 0.09 | 0.02 | 0.15 | 0.03 | **0.01** | |

### GENERAL EXPERIMENTAL PROCEDURES

### Example 1

### Method for Genotyping of the Aldose Reductase A-C Repeat

### Sequence Polymorphism.

Genomic DNA is isolated from whole blood using the QIAamp® 96 DNA Blood Kit (QIAGEN, Valencia, CA). Approximately 100 ng of genomic DNA is used in each 50 µl PCR reaction containing 1X PCR Buffer II (Applied Biosystems, Foster City, CA), 0.2 mM MgCl₂, 0.2 µM of each primer, and 2.5 units of AmpliTaq Gold (Applied Biosystems). The PCR primer sequences used are 5'-GAATCTTAACATGCTCTGAACC-3' (5'-end labeled with the fluorescent dye 6-FAM) for the sense primer and 5'-GCCCAGCCCTATACCTAGT-3' for the antisense primer (described in Shah *et al.* 1998). PCR cycling parameters for the DNA Engine Tetrad Thermal Cycler (MJ Research, Waltham, MA) are an initial denaturation at 95° C for 9 minutes, followed by 35 cycles of denaturation at 95° C for 30 seconds/annealing at 61° C for 30 seconds/extension at 72° C for 30 seconds, and a final extension at 72° C for 3 minutes. Samples are prepared for gel loading by combining 2 µl of TAMRA-500™ internal lane standard (Applied Biosystems), 1 µl of 1:4 diluted PCR product, and 5 µl of 5:1 deionized formamide:blue dextran loading dye. A volume of 1 µl of this mixture is loaded on a 5% urea-polyacrylamide gel and electrophoresed using ABI 377 DNA Sequencer (Applied Biosystems). A calibration curve is created based on the TAMRA-500 internal standard for each lane, and the unknown PCR product length is calculated using GENESCAN® software (Applied Biosystems). PCR product lengths are then translated into the applicable allele, as shown below. The genotypes are written as "allele1/allele2" (e.g., Z/Z-2).

| **PCR Product Length** | **Allele** |
|---|---|
| 142 | Z+4 |
| 140 | Z+2 |
| 138 | Z |
| 136 | Z-2 |
| 134 | Z-4 |
| 132 | Z-6 |

### Example 2

### Evaluation of Neuroelectrophysiology

### Definitions of terms used in this example:

Amplitude is the measurement of the size (height) of the Compound Action Potential or M-wave, reflecting the number of neurons or motor units firing and the synchrony of activity measured in millivolts (mv) or microvolts (µv) from baseline to the peak of the response.

Compound Action Potential is the summation of action potentials from neurons forming a peripheral nerve. For mixed nerves, this includes sensory and motor fibers.

Latency is defined as the interval between the onset of a stimulus and the onset of a neural or motor response, measured in milliseconds. All Latency measurements are made from the onset of stimulation to the onset of the initial negative deflection in the evoked response (i.e., onset of depolarization). Latency measurements are assessed using a Computer Cursor (i.e., movable sprite within the applicable computer program that can be used to mark the Latency or Amplitude of a response) and are recorded to the nearest 0.1 msec. All Amplitudes are measured from the baseline (pre-stimulus, if available) to the peak of the negativity. Amplitude measurements are assessed using a Computer Cursor and are recorded to the nearest 0.1 µv for sensory responses and 0.1 mv for motor responses. All distances are measured in millimeters and recorded to the nearest 1.0 mm.

M-wave is the compound response from motor units within a muscle.

Nerve Conduction Velocity ("NCV") is defined as the maximal speed in which Compound Action Potentials are propagated within a nerve, generally reflecting the activity in the fastest and largest neurons and calculated in meters per second by dividing distance in mm by Latency. Motor NCV is defined as the distance between the stimulating cathodes divided by the Latency difference to the onset of the M-wave response following stimulation at a proximal and distal site. Proximal sensory NCV is defined as the distance between the stimulating cathodes divided by the Latency difference to the onset of initial negative component following stimulation at a proximal and distal site. Distal sensory NCV is defined by dividing the distance between the distal stimulating cathode and the active recording electrode by the absolute Latency of the initial negative component of the distal sensory response.

### Equipment

1. Electromyogram (EMG) machine with the following features:
   a. Two channel differential amplification
   b. Averaging capability
   c. Internal cursor for time and Amplitude measurements
   d. Stimulus isolation unit for generation of electrical pulses
2. Digital thermometer with surface probes, accurate to 0.1 °C.
3. Equipment capable of raising limb temperature 5.0°C in 15 min. This may be accomplished using either a dry or wet heat blanket, a water bath, or a radiant lamp.

### Motor Nerve Measurements

The following motor nerve measurements are performed orthodromic - using conduction in the direction in which the impulse is normally conveyed - i.e., sensory towards the body, motor away from the body.
1. Median Motor
   a. NCV of forearm segment
   b. M-wave Amplitude (abductor pollicis brevis)
2. Peroneal Motor
   a. NCV of distal segment (knee to ankle)
   b. M-wave Amplitude (extensor digitorum brevis)
   c. absolute Latency of the F-wave response

### Sensory Nerve Measurements

The following sensory nerve measurements are performed antidromic -- using conduction in the direction opposite to that in which the impulse is normally conveyed:
3. Median Sensory
   a. NCV of forearm segment
   b. NCV of distal segment (wrist to interdigital cleft)
   c. Amplitude of compound sensory response for distal stimulation (initial depolarization at index finger)
4. Ulnar Sensory
   a. NCV from wrist to interdigital cleft
   b. Amplitude of compound sensory response (initial depolarization at fifth finger)
5. Ratio of NCV for distal ulnar sensory (wrist to digit) divided by NCV for distal median sensory (wrist to digit)
6. Sural Nerve
   a. NCV segment from mid-calf to ankle
   b. Amplitude of compound sensory response (initial depolarization at the inferior aspect of the lateral malleolus).

### Methods

1. All electrophysiological procedures are performed unilaterally and on the same side for both upper and lower limbs. The non-dominant limb is used unless contraindicated by localized pathology (e.g., injuries, history of entrapments, or surgery).
2. Electrodes. All stimulating and recording electrodes are applied to the skin surface. Ring electrodes, which encircle the finger, are preferred for median sensory nerve.
   a. The skin is cleaned with a suitable solvent, e.g., alcohol, acetone.
   b. The skin is lightly abraded with electrode paste.
   c. A conducting medium is applied, e.g., electrode jelly, between the electrode and the skin.
3. Skin temperature control. Skin temperature is maintained at 33° C for the upper limb and 32° C for the lower limb, plus or minus 2° C throughout testing. Skin temperature is measured prior to and at the conclusion of testing at sites midway between the stimulating and recording electrodes for each limb. Temperature is monitored and adjusted during testing using either a feedback controlled infrared radiant heater, a water bath or a temperature controlled blanket wrap.
4. Averaging. All sensory measurements are taken from a computer averaged signal using internal cursors (i.e., having a function that enables the user to determine the exact time and amplitude of each point along the generated wave form). This averaging technique enhances the signal to noise ratio and facilitate accurate measurement of response onset; 3 to 20 stimuli are averaged. When measuring the M-wave response, single reproducible sweeps may be adequate; however, averaging of 3 to 5 stimuli can significantly enhance onset resolution.
5. Stimulation. All testing is done with the subject carefully isolated from ground using a professional stimulus isolation unit. Stimulus intensity varies as a function of the specific nerve and site of stimulation; the intensity is adjusted according to the guidelines below. Stimulus duration is between 0.05 and 0.5 msec. Stimulus rate is approximately 1/sec.
6. Specific Nerves:
   a. Median Motor
      1) The active recording electrode is placed over the endplate area of the abductor pollicis brevis.
      2) The reference on the index finger is placed at least 3.0 cm distal to the active lead.
      3) The ground is placed on the back of the hand.
      4) When stimulating at the wrist, position the stimulating cathode over the median nerve 2.0 cm proximal to the distal wrist crease. For best results, the electrodes are positioned between the P. longus and F. carpi radialis tendons. There should be a minimal separation of 2.0 cm between the anode and cathode, and the anode should be proximal to the cathode.
      5) When stimulating at the elbow, the stimulating cathode is positioned over the median nerve immediately distal to the elbow crease.
      6) Stimulus intensity is adjusted (along with duration) to elicit a supramaximal M-wave response.
   b. Peroneal Motor
      1) The active recording electrode is placed over the endplate area of the extensor digitorum brevis.
      2) The reference is placed on the lateral surface of the ipsilateral foot at the base of the fifth digit.
      3) The ground is placed on the mid-line at the level of the ankle.
      4) When stimulating at the ankle, the cathode is positioned over the peroneal nerve 8.0 cm proximal to the active recording electrode.
      5) When stimulating at the knee, the cathode is positioned overlying the peroneal nerve, slightly distal and lateral to the head of the fibula.
      6) Stimulus intensity is adjusted (along with duration) to elicit a supramaximal for M-wave response.
   c. Median Sensory
      1) The active ring electrode is positioned on the index finger, 1.0 cm distal to the interdigital cleft.
      2) The reference electrode is positioned on the same index finger 1.0 cm distal to the distal interphalangeal joint.
      3) The ground is placed between the active electrode and the point of stimulation.
      4) When stimulating at the wrist, the stimulating cathode is positioned over the median nerve 2.0 cm proximal to the distal wrist crease. For best results, the electrodes are positioned between the P. longus and the F. carpi radialis tendons. There should be a minimal separation of 2.0 cm between the anode and cathode, and the anode should be proximal to the cathode.
      5) When stimulating at the elbow, the stimulating cathode is positioned over the median nerve immediately distal to the elbow crease.
      6) Stimulus intensity is adjusted (along with duration) to elicit supramaximal sensory negativity.
   d. Ulnar Sensory
      1) The active ring electrode is positioned on the fifth digit, 1.0 cm distal to the interdigital cleft.
      2) The reference electrode is positioned on the same finger 1.0 cm distal to the distal interphalangeal joint.
      3) The ground is placed between the active electrode and the point of stimulation.
      4) The stimulating cathode is placed over the flexor carpi ulnaris tendon, approximately 14 cm proximal to the active recording site.
      5) Stimulus intensity is supramaximal for the sensory negativity.
   e. Sural Sensory
      1) Place the active electrode over the sural nerve at the level of the lower tip of the lateral malleolus.
      2) Place the reference on the lateral surface of the same foot at least 3.0 cm distal to the active electrode.
      3) Position the ground on the lower calf, between the stimulating and recording electrodes.
      4) Position the stimulating cathode approximately 14.0 cm proximal to the active electrode along the dorsal mid-calf.
      5) Stimulus intensity is supramaximal for the sensory negativity.

### Additional Technical Considerations

1. The Amplitude of all sensory and motor responses must be supramaximal. It is necessary to determine that increasing the intensity of stimulation does not further increase the Amplitude of the evoked response.
2. The waveform must be measured using appropriate voltage and time windows. If the signal is small, the gain setting should be increased so that the waveform occupies approximately 50% of the viewing window. The onset of a M-wave response should be measured using a high gain setting that "clips" the peak of the M-wave.
3. The M-wave associated with stimulation of the proximal site should match the Amplitude and waveform of that evoked by distal stimulation (within 20% maximal Amplitude).
4. The impedance of the recording and stimulating electrodes and the location and type of ground should be selected to reduce the stimulus artifact so that a true baseline measure can be determined. The stimulus artifact is the electrical signal generated directly by the stimulating current beginning at time zero and continuing for several milliseconds due to amplifier saturation, a possible source of interference with the sensory potentials.
5. The ideal distance between the stimulating cathode and the active recording electrode for sural nerve recording is 14.0 cm.
6. Differential amplification involving amplification of only the difference in the activity recorded at two sites may be used to minimize noise and enhance the signal. The active electrode is placed overlying the targeted site (belly of the muscle, center of the nerve) while the second, reference electrode is placed overlying a nearby inactive site.

## Claims

1. A characterization method comprising:
determining the value of n of the (A-C)ₙ repeat Z sequence associated with each allele of the aldose reductase gene of a subject; and
characterizing said subject as having the attribute of preferentially benefiting from the administration of an agent for prevention or treatment of a disease or pathological condition that is mediated by having at least one allele of said Z sequence wherein the value of n is less than 24, with the proviso that the value of n of at least one allele of said subject is determined to be less than 24.

2. A method of claim 1 wherein said subject has a disease or pathological condition that is mediated by having at least one allele of said Z sequence wherein n is less than 24.

3. A characterization method comprising:
determining the value of n of the (A-C)ₙ repeat Z sequence associated with each allele of the aldose reductase gene of a subject; and
characterizing said subject as having the attribute of being likely to develop a disease or pathological condition that is mediated by having at least one allele of said Z sequence wherein the value of n is less than 24, with the proviso that the value of n of at least one allele of said subject is determined to be less than 24.

4. A method of claim 3 wherein said subject has diabetes mellitus.

5. Use of an agent for treatment or prevention of a disease or pathological condition that is mediated by having at least one allele of the (A-C)ₙ repeat Z sequence associated with the aldose reductase gene wherein the value of n is less than 24, for the manufacture of a medicament for the treatment or prevention of complications associated with diabetes mellitus in a subject, wherein said subject has been characterized as having at least one allele of said Z sequence wherein n is less than 24.

6. A use of claim 5 wherein said subject is further characterized as having diabetes mellitus.

7. A method of claim 1 or a use of claim 5 with the further proviso that the values of n of both alleles of said subject are determined to be less than 24.

8. A use of claim 5 wherein said subject has two alleles of said Z sequence wherein n is less than 24 and said disease or pathological condition is mediated by having two alleles wherein n is less than 24.

9. A method of claim 1 or a use of claim 5 wherein said agent is selected from insulin, an insulin secretion stimulating sulfonylurea compound, a glycogen phosphorylase inhibitor (GPI), a biguanide hepatic glucose output inhibitor, a thiazolidinedione antidiabetic agent, an alpha-glucosidase inhibitor, a protein tyrosine phosphatase-1B (PTP-1B) inhibitor, a dipeptidyl peptidase IV (DPPIV) inhibitor, a glycogen synthase kinase 3 beta (GSK-3β) inhibitor, a peroxisome proliferator-activated receptor gamma (PPARγ) agonist and a glucagon receptor antagonist.

10. A method of claim 1 or a use of claim 5 wherein said agent is selected from a selective serotonin reuptake inhibitor (SSRI), a 3-hydroxy-3-methylglutaryl coenzyme A reductase inhibitor (a statin), a γ-aminobutyric acid (GABA) agonist, an angiotensin converting enzyme (ACE) inhibitor, an angiotensin-II (A-II) receptor antagonist, a phosphodiesterase type 5 (PDE-5) inhibitor and a polyol pathway inhibitor.

11. A method of claim 1 or a use of claim 5 wherein said agent is a polyol pathway inhibitor.

12. A method of claim 1 or 3 wherein the characterizing of said subject comprises recording the identity and said attribute of said subject in an information recording media.

13. A method or use of any one claim of this invention wherein said disease or pathological condition is a complication related to diabetes mellitus.

14. A method or use of any one claim of this invention wherein said subject is a mammal.

15. A method or use of any one claim of this invention wherein said subject is a human.
